(19) Europäisches Patentamt
European Patent Office
Office européen des brevets

(11) EP 0 657 040 B1

# (12) EUROPÄISCHE PATENTSCHRIFT

(45) Veröffentlichungstag und Bekanntmachung des Hinweises auf die Patenterteilung:
**26.04.2000 Patentblatt 2000/17**

(21) Anmeldenummer: **93915862.2**

(22) Anmeldetag: **09.07.1993**

(51) Int. Cl.7: **G02F 1/35**, C07C 403/12, C07C 403/24

(86) Internationale Anmeldenummer:
**PCT/EP93/01798**

(87) Internationale Veröffentlichungsnummer:
**WO 94/02881 (03.02.1994 Gazette 1994/04)**

## (54) POLYENE MIT NICHTLINEAROPTISCHEN EIGENSCHAFTEN

POLYENES WITH NON-LINEAR OPTICAL PROPERTIES

POLYENES A PROPRIETES OPTIQUES NON LINEAIRES

(84) Benannte Vertragsstaaten:
**DE FR GB**

(30) Priorität: **21.07.1992 DE 4224015**

(43) Veröffentlichungstag der Anmeldung:
**14.06.1995 Patentblatt 1995/24**

(73) Patentinhaber:
**BASF Aktiengesellschaft**
**67063 Ludwigshafen (DE)**

(72) Erfinder:
- **FISCH, Herbert**
  **D-6706 Wachenheim (DE)**
- **HAAS, Karl-Heinz**
  **D-6710 Frankenthal (DE)**
- **SCHROF, Wolfgang**
  **D-6719 Neuleiningen (DE)**
- **PAUST, Joachim**
  **D-6708 Neuhofen (DE)**
- **TICKTIN, Anton**
  **D-6900 Heidelberg (DE)**
- **KRAUSE, Wolfgang**
  **D-6800 Mannheim 81 (DE)**

(56) Entgegenhaltungen:
**EP-A- 0 005 748** **EP-A- 0 455 119**

- **PROCEEDINGS OF THE SPIE (5TH CONFERENCE ON NONLINEAR OPTICAL PROPERTIES OF ORGANIC MATERIALS SAN DIEGO,CA,USA, 22-24 JULY 1992 Bd. 1775 , 1993 , USA Seiten 349 - 358 A.ESSER,H.FISCH 'Nonlinear Optics of Astaxanthin thin films'**
- **ELECTRONICS AND COMMUNICATIONS IN JAPAN,PART 2 ELECTRONICS Bd. 75, Nr. 12 , Dezember 1992 , USA Seiten 555 - 560 S.MUTO,K.MIYAGAWA 'Hybrid- type Optical Bistability in a Thin-Film Polymer Waveguide Doped with nonlinear Organic Dyes'**
- **JOURNAL OF CHEMICAL PHYSICS Bd. 98, Nr. 4 , 15. Februar 1993 , USA Seiten 2524 - 2533 M.E.ORCZYK 'dynamics of third order nonlinearity of canthaxanthin carotenoid......'**
- **APPL.PHYS.LETTERS Bd. 23, Nr. 4 , 15. August 1973 , USA Seiten 178 - 180 J.P.HERMANN 'optical nonlinearities in conjugated systems : beta-carotene'**
- **PROCEEDINGS OF THE SPIE(4TH CONFERENCE ON NONLINEAR OPTICAL PROPERTIES OF ORGANIC MATERIALS ,SAN DIEGO,CA,24-26 JULY 1991 Bd. 1560 , 1991 , USA Seiten 172 - 182 A.GIERULSKI 'frequency measurements of chi.3 ......'**
- **JOURNAL OF CHEMICAL PHYSICS Bd. 95, Nr. 9 , 1. November 1991 , USA Seiten 6400 - 6412 J.B.VAN BEEK 'resonant third harmonic generation in all-trans beta-carotene'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.7 (HRS)/3.6

## Beschreibung

Die vorliegende Erfindung betrifft Polyene der allgemeinen Formel I

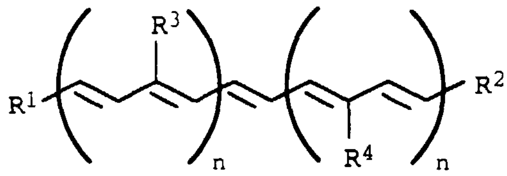

(I)

worin $R^1$ und $R^2$ gleich oder verschieden voneinander sein können und Wasserstoff, $C_6$- bis $C_{22}$-Aryl, $C_5$- bis $C_{10}$-Heteroaryl, $C_4$- bis $C_{30}$-Cycloalkyl, $C_4$- bis $C_{30}$-Cycloalkenyl, $C_6$- bis $C_{10}$-Cycloalkenon, wobei die vorgenannten Reste jeweils auch einen oder mehrere Substituenten ausgewählt aus der Gruppe der $C_1$- bis $C_{30}$-Alkyl-, $C_2$- bis $C_{30}$-Alkenyl-, $C_1$- bis $C_{30}$-Alkoxy-, $C_4$- bis $C_{30}$-Cycloalkyl-, $C_4$- bis $C_{30}$-Cycloalkenyl-, $C_6$- bis $C_{18}$-Arylreste, Halogen, -OH, -COOH, -CHO, -$COOR^5$, CN, -$NH_2$, -$NHR^6$, -$NR^7R^8$ und $NO_2$ haben können, -CHO, -COOH, -$COOR^9$ oder einen Rest der allgemeinen Formel

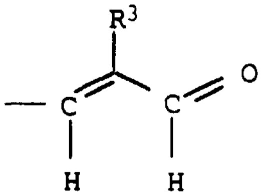

,

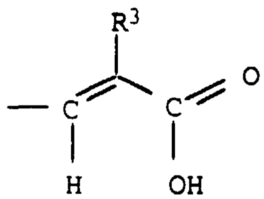

oder

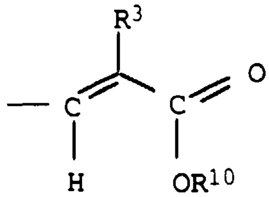

bedeuten und $R^3$ und $R^4$ gleich oder verschieden voneinander sein können und Wasserstoff, $C_1$- bis $C_{30}$-Alkyl, $C_1$- bis $C_{30}$-Alkoxy, -SH, -SSH, -$NH_2$, -$NR^{11}R^{12}$ bedeuten, wobei die Reste $R^5$ bis $R^{12}$ gleich oder verschieden und unabhängig voneinander $C_1$- bis $C_{30}$-Alkyl, $C_2$- bis $C_{30}$-Alkenyl, $C_4$- bis $C_{30}$-Cycloalkyl, $C_4$- bis $C_{30}$-Cycloalkenyl, $C_6$- bis $C_{18}$-Aryl sein können und n eine ganze Zahl von 3 bis 10 darstellt.

Darüber hinaus betrifft die vorliegende Erfindung deren Verwendung als nichtlinearoptische Materialien.

Die Synthese von Polyenen mit carotinoiden Strukturen ist seit langem bekannt (s. z.B.: O. Issler, Birkhäuser Verlag Basel u. Stuttgart, 1971, S. 73ff).

Es ist bereits bekannt, daß konjugierte organische Materialien hohe nichtlinearoptische (NLO) Effekte erbringen. Bedingt wird diese Tatsache durch die leichte Polarisierbarkeit der delokalisierten $\pi$-Elektronen in diesen Systemen. Dabei hat die Anzahl der Doppelbindungen einen entscheidenden Einfluß auf die nichtlinear optischen Eigenschaften von konjugierten Polyenen. So weist z.B. trans-Retinol mit 5 konjugierten Doppelbindungen schlechtere nichtlinearoptische Koeffizienten 3. Ordnung auf, als trans-$\beta$-Carotin mit 7 konjugierten Doppelbindungen (H. Nakanishi, Nonlinear Optics, 1991, 1, 223-236).

Die nichtlineare Optik beschäftigt sich ganz allgemein mit der Wechselwirkung elektromagnetischer Felder und Materie und kann zu einem Brechungsindex fuhren, der von der Lichtintensität abhängig ist. R.N. Prasad und D.J. Williams geben in dem Buch "Introduction to nonlinear optical effects in molecules and polymers (Wiley 1991)" ausführliche Information zu dieser Thematik.

Zusammengefaßt ist das nachstehende von Bedeutung: Ganz allgemein emittiert ein Stoff Licht, wenn in ihm Dipole schwingen, wobei die Frequenz der emittierten Lichtwelle gleich der Schwingungsfrequenz der Dipole ist. Enthalten die schwingenden Dipole mehrere Frequenzkomponenten, kommen diese allein in dem vom betreffenden Stoff emittierten Licht vor. Sofern die räumliche Ausdehnung des Stoffes größer als die Wellenlänge des emittierten Lichts ist, sollten die im Stoff schwingenden identischen Dipole möglichst in gleicher Richtung und mit einer Phasendifferenz schwingen, die dafür sorgt, daß das von einem Volumenelement emittierte Licht nicht durch destruktive Interferenz mit dem von einem anderen Volumenelement emittierten Licht ausgelöscht wird.

In einem polarisierbaren Stoff wird durch ein von außen angelegtes Feld E eine makroskopische Polarisation bewirkt, welche als Dipolmoment pro Volumeneinheit definiert ist.

Enthält das polarisierbare Material keine permanenten molekularen Dipole, resultiert das Dipolmoment und damit die makroskopische Polarisation P aus der Verschiebung der Elektronen um den Betrag d aus ihrer Ruhelage, d.h. dem Schwerpunkt der positiven Ladung heraus. Sind dagegen permanente Dipole enthalten, ändert sich durch das angelegte Feld E das permanente Dipolmoment nach demselben Mechanismus.

Solange die Verschiebung d proportional zum elektrischen Feld bleibt, ist auch die Polarisation P proportional zum elektrischen Feld E, was in der bekannten linearen Gleichung 1

$$P = \varepsilon_0 * \chi * E \qquad \text{(Gl. 1)}$$

zum Ausdruck kommt. In der Gleichung bezeichnet $\varepsilon_0$ die absolute Dielektrizitätszahl und $\chi$ die dielektrische Suszeptibilität.

Wird das von außen angelegte Feld verstärkt, muß naturgemäß jeder Stoff oberhalb einer für ihn spezifischen Feldstärke eine Abweichung vom linearen Gesetz gemäß der Gleichung 1 zeigen. Das mechanische Analogon ist die Abweichung vom Hookschen Gesetz bei Überbelastung einer Feder. Solche Abweichungen von der Linearität werden mathematisch durch Entwicklung in eine Potenzreihe behandelt, d.h. man entwickelt die nichtlineare Funktion nach Potenzen der Variablen E, woraus Gleichung 2, die grundlegende Gleichung der nichtlinearen Optik resultiert.

$$P = \varepsilon_0 * (\chi^{(1)} * E + \chi^{(2)} * E^2 + \chi^{(3)} * E^3) \qquad \text{(Gl. 2)}$$

Dabei bedeutet:

$\chi^{(1)}$ die dielektrische Suszeptibilität erster Ordnung, welche letztlich für lineares Verhalten des betreffenden Materials verantwortlich ist, d.h. Brechungsindex und Absorptionskonstante sind feldunabhängig,

$\chi^{(2)}$ die dielektrische Suszeptibilität zweiter Ordnung, welche das nichtlinearoptische Verhalten zweiter Ordnung des betreffenden Stoffs verantwortlich ist,

und $\chi^{(3)}$ die dielektrische Suszeptibilität dritter Ordnung, auf welche das nichtlinearoptische Verhalten dritter Ordnung des betreffenden Stoffes zurückgeht.

$\chi^{(2)}$ bzw. $\chi^{(3)}$ sind Materialkonstanten, die von der Molekülstruktur, von der Kristallstruktur, von der Frequenz des Lichts und im allgemeinen von der Temperatur abhängig sind. Sie können bekannterweise mit Hilfe der Frequenzverdoppelung (SHG) oder der Bestimmung des Pockels-Effektes ($\chi^{(2)}$) oder der "Vier-Wellen-Mischmethode" (Degenerate Four Wave Mixing, DFWM) oder dem Frequenzverdreifachungsexperiment (Third Harmonie Generation, THG; $\chi^{(3)}$) ermittelt werden. Diese Methoden werden im Buch von Prasad und Williams: Introduction to nonlinear optical effects in molecules and polymers, Wiley 1991, grundsätzlich beschrieben.

Stoffe mit einer nichtverschwindenden dielektrischen Suszeptibilität $\chi^{(3)}$ besitzen einen intensitätsabhängigen Brechungsindex und eignen sich unter anderem zur Herstellung rein optischer Schalter und damit zur Verwendung in optisch arbeitenden Computern. Weitere Anwendungsmöglichkeiten für derartige Materialien werden von D.-R. Ulrich unter dem Titel "Nonlinear Optical Polymer Systems and Devices" in Molecular Crystals and Devices, Band 180, Seiten 1 bis 31, 1988, beschrieben.

Für die Anwendung von nichtlinear optischen Materialien (hier: Materialien, die auf einem hohen $\chi^{(3)}$ basieren) in optischen Bauelementen ist eine Kombination von verschiedenen Stoffeigenschaften notwendig. So genügt ein hohes $\chi^{(3)}$ allein keineswegs den Anforderungen an ein optisches Bauteil. Daneben sollte die Relaxation und damit die Schaltzeit sehr klein sein (im Bereich von Femtosekunden), die optischen Verluste durch Absorption, Streuung etc. sollten minimal sein und zuletzt sollte eine gute Verarbeitbarkeit der Materialien in Form von Schichten z.B. in optischen Lichtleitern gegeben sein.

Die derzeitige Entwicklung von $\chi^{(3)}$-Materialien wird schwerpunktmäßig bei der Optimierung des Betrages von $\chi^{(3)}$ und der Relaxationszeit durchgeführt, wobei jedoch die Fragen einer guten Verarbeitbarkeit bei kleinen optischen Verlusten eine immer größere Rolle spielen.

In diesem Zusammenhang sind vor allem Polymere die ausgedehnte konjugierte $\pi$-Systeme aufweisen z.B. Polydiacetyle, Polythiophene oder auch Polyphenylenvinylidene (s. auch: D.R. Ulrich, Polymers tor nonlinear optics, Mol. Cryst. Liq. Cryst. 1990, 189, 3-38). Diese polymeren Werkstoffe bieten im Gegensatz zu niedermolekularen Stoffen mit NLO-Eigenschatten, den Vorteil guter mechanischer Eigenschaften und besserer Verarbeitbarkeit.

Als Alternative bieten transparente organische Polymere ohne spezielle nichtlinearoptische Eingeschaften die Möglichkeit nichtlinearoptisch aktive niedermolekulare Stoffe einzulagern, womit ebenfalls eine gute Verarbeitbarkeit bzw. mechanische Beanspruchbarkeit der Materialien gewährleistet ist. Zusätzlich kann in diesem Fall das Matrixpolymer Schutzfunktionen, die zur chemischen Stabilisierung des eingelagerten NLO-Materials ausgenutzt werden können, übernehmen.

Die Applikation der Materialien erfolgt im allgemeinen mittels verschiedener Dünnschichttechniken, die in der Elektronikindustrie zur Herstellung von dünnen Schichten auf verschiedenen Substraten Anwendung finden. Ein relativ einfaches Verfahren zur Herstellung von Schichten für optische Materialien bietet das sogenannte Spin-Coat-Verfahren. Diese Methode führt zu homogenen, transparenten Schichten, die entweder aus reinen nichtlinearoptisch aktiven Materialien oder einer Mischung aus transparentem Matrixpolymer und NLO-Material aufgebaut sind. Zur technischen Realisierung dieses Verfahrens müssen die zu beschichtenden Substanzen entweder rein oder in Kombination mit einem Polymer homogen gelöst werden. Die Schichtdicke der beim Spincoatprozeß erhaltenen Schicht ist abhängig von den rheologischen Eigenschaften der Lösungen, sowie von der Umdrehungsgeschwindigkeit des Substrates bei der Beschichtung.

Zur Herstellung von Filmen mit Schichtdicken > 1 µm können auch Rakel- bzw. Gießtechniken angewendet werden. Nach Abschluß der Beschichtung muß auf sorgfältige Trocknung geachtet werden, um Rißbildung bzw. Inhomogenitäten zu unterbinden.

Die Aufgabe der vorliegenden Erfindung ist die Synthese von höheren Homologen der Verbindungsklasse der Carotinoide (Anzahl der konjugierten Doppelbindungen ≥ 13), die Übertragung des Syntheseprinzips auf andere nicht carotinoide Polyene definierter Konjugationslänge und deren Einsatz als optoelektronische Materialien.

Diese Aufgabe wird von den eingangs definierten Polyenen der allgemeinen Formel I

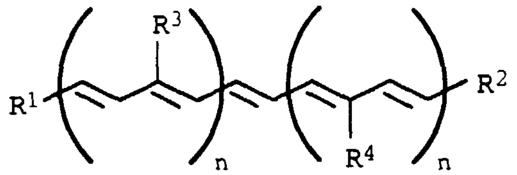 (I)

gelöst. Die endständigen Reste $R^1$ und $R^2$ können gleich oder verschieden voneinander sein. $R^1$ und $R^2$ können Wasserstoff bedeuten oder einen $C_6$- bis $C_{22}$-Arylrest, insbesondere Phenyl, aber auch einen $C_5$- bis $C_{10}$-Heteroarylrest, beispielsweise Pyridyl oder Thienyl. Darüber hinaus können $R^1$ und $R^2$ auch einen $C_4$- bis $C_{30}$-Cycloalkylrest wie Cyclopentyl, Cyclohexyl oder Cyclododecyl darstellen. $R^1$ und $R^2$ können des weiteren ein $C_4$- bis $C_{30}$-Cycloalkenyl wie Cyclohexenyl oder Cyclooctenyl sein. Vorzugsweise sind $R^1$ und $R^2$ ein $C_6$- bis $C_{10}$-Cycloalkenonrest, darunter Cyclohexenon, Cycloheptenon, Cyclooctenon, Cyclodecenon oder Cyclododecenon.

Die vorgenannten Reste können sowohl unsubstituiert sein, als auch einen oder mehrere Substituenten haben. Zu den Substituenten zählen $C_1$- bis $C_{30}$-Alkylreste, z.B. Methyl, Ethyl, n-Propyl, i-Butyl, neo-Pentyl oder $C_2$- bis $C_{30}$-Alkenylreste wie Ethenyl, n-1-Pentenyl, n-4-Octenyl, n-9,12-Docasadienyl oder 10-Eicosenyl. Weiterhin können $C_1$- bis $C_{30}$-Alkoxygruppen, beispielsweise Methoxy, Ethoxy, n-Butoxy oder n-Decyloxy geeignete Substituenten sein.

Ebenso zählen $C_4$- bis $C_{30}$-Cycloalkyl wie Cyclopentyl, Cyclohexyl oder Cycloheptyl sowie $C_4$- bis $C_{30}$-Cycloalkenylreste, beispielsweise Cyclopentenyl, Cyclohexenyl oder Cyclooctenyl zu den erfindungsgemäßen Substituenten. Ferner können $C_6$- bis $C_{18}$-Arylreste wie Phenyl, Biphenyl oder Naphthyl Substituenten sein. Außerdem zählen zu den Substituenten Halogenatome, darunter Chlor und Brom, sowie die funktionellen Gruppen -OH, -COOH, -CHO, $-COOR^5$, CN, $-NH_2$, $-NHR^6$, $-NR^7R^8$ und $NO_2$. Beispiele für substituierte Reste $R^1$ und $R^2$ sind

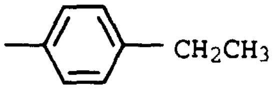

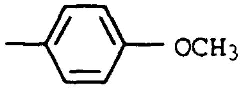

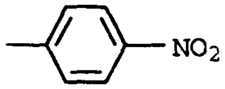

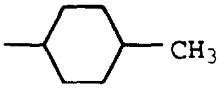

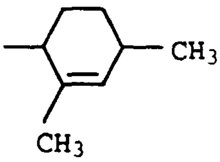

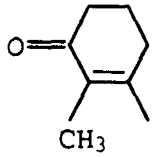

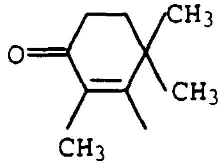

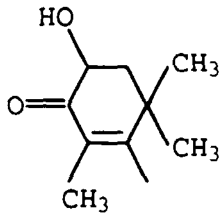

Darunter sind die substituierten Cyclohexenonreste besonders bevorzugt.

Die endständigen Reste $R^1$ und $R^2$ können daneben auch die funktionellen Gruppen -CHO, -COOH oder -$COOR^9$ weiterhin können $R^1$ und $R^2$ auch für folgende ungesättigte Aldehyd-, Säure- oder Estergruppen stehen:

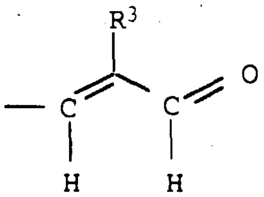 , 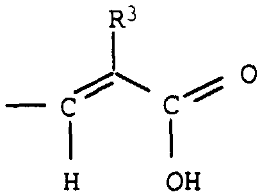 oder 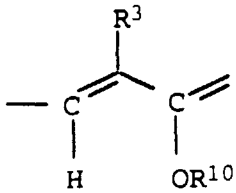

Die Reste $R^3$ und $R^4$ können gleich oder verschieden voneinander sein und Wasserstoff, $C_1$- bis $C_{30}$-Alkyl wie Methyl, Ethyl, n-Propyl, n-Butyl oder i-Butyl, $C_1$- bis $C_{30}$-Alkoxy, beispielsweise Methoxy, Ethoxy, n-Butoxy oder i-Butoxy bedeuten. Erfindungsgemäß können $R^3$ und $R^4$ auch für die Gruppen -SH, -SSH, -$NH_2$ oder -$NR^{11}R^{12}$ stehen. Besonders bevorzugte Reste $R^3$ und $R^4$ sind Wasserstoff, Methyl, Propyl oder Isopropyl.

Die Reste $R^5$ bis $R^{12}$ können gleich oder verschieden und unabhängig voneinander $C_1$- bis $C_{30}$-Alkyl wie Methyl, Ethyl, n-Propyl, i-Propyl oder t-Butyl, $C_2$- bis $C_{30}$-Alkenyl, beispielsweise Ethenyl, Propenyl, n-1-Pentenyl oder n-1-Octenyl bedeuten. Weiterhin können $R^5$ bis $R^{11}$ für $C_4$- bis $C_{30}$-Cycloalkyl, darunter Cyclopentyl, Cyclohexyl oder Cyclooctyl oder für $C_4$- bis $C_{30}$-Cycloalkenyl, z.B. Cyclohexenyl oder Cyclooctenyl stehen. Ebenso können $R^5$ bis $R^{12}$ einen $C_6$- bis $C_{18}$-Arylrest, wie Phenyl oder Naphthyl, bedeuten.

Erfindungsgemäß ist n eine ganze Zahl von 3 bis 10, bevorzugt 3 bis 8.

Die Synthese der erfindungsgemäßen Polyene kann grundsätzlich nach verschiedenen Syntheseprinzipien erfolgen. Eine spezielle Zugangsmöglichkeit stellt die Carbonylolefinierung dar, bei der Ylide mit Carbonylverbindungen umgesetzt werden. Beispiele hierfür sind die Wittigreaktion oder Wittig analoge Reaktionen wie die Variante nach Horner. Durch die Wahl der Reaktionsparameter, beispielsweise des verwendeten Ylides kann der regio- bzw. stereochemische Verlauf der Reaktion beeinflußt werden. Derartige Reaktionen sind an sich bekannt. Ausführliche Informationen zu der Carbonylolefinierung können beispielsweise Tietze, Eicher: Reaktionen und Synthesen, Thieme Verlag oder J.

March: Advanced Organic Chemistry, McGraw-Hill entnommen werden.

Die erfindungsgemäßen Polyene können beispielsweise dadurch hergestellt werden, daß Alkene, die mit $R^3$ und $R^4$ substituiert sind, deren Anzahl von Doppelbindungen m kleiner als n ist und die eine oder zwei endständige Aldehyde und/oder Ketogruppen aufweisen einer Carbonylolefinierungsreaktion unterworfen werden.

Die Synthese der erfindungsgemäßen Polyene kann beispielsweise durch Umsetzen von Triphenylphosphoniumbromiden II mit Alkenen III in Gegenwart einer Base umgesetzt werden:

Schema 1:

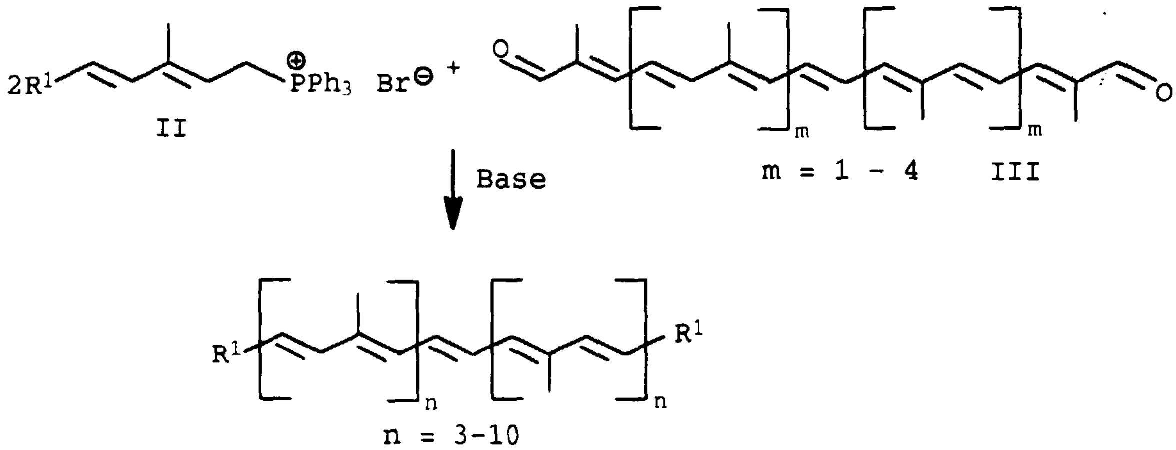


Als Base kommt beispielsweise Natriumamid oder n-Butyllithium in Betracht.

Diese Herstellungsmethodik erlaubt, ausgehend von definierten Polyen-Bausteinen, die Einstellung definierter Konjugationslängen sowie die Synthese von Polyene mit hohen all-trans Anteilen. Die Löslichkeit der Polyene läßt sich durch die Wahl der Reste $R^1$ bis $R^4$ beeinflussen. So können beispielsweise Reste wie Isopropyl oder Ethyl die Löslichkeit der Polyene in N-Methylpyrrolidon oder Chloroform erhöhen.

Die synthetisierten Materialien können in Form von Lösung direkt dem oben beschriebenen Spin-Coat-Verfahren unterzogen werden. Der Feststoffgehalt der Lösungen liegt dabei in der Regel im Bereich von 0,5 bis 50 Gew.-%, vorzugsweise von 0,5 bis 15 Gew.-%.

Den erfindungsgemäßen Polyenen können transparente organische Polymere beigemischt sein. Dabei kommen im Prinzip alle Polymere in Frage, die transparente Filme liefern. Geeignete Polymermaterialien sind im allgemeinen Polystyrol, Polyvinylacetat, Polyacrylate, nichtkristalline Polyurethane, Mischpolyamide, Polyimidazole, vorzugsweise jedoch Polymethylmethacrylat oder Polyvinylpyrrolidon. Der Gehalt an NLO-aktivem Polyen in der Mischung beträgt im allgemeinen 5 bis 95 Gew.-%, vorzugsweise 10 bis 80 Gew.-%.

Die Wahl geeigneter Lösungsmittel für den Spin-Coat-Prozeß ist hauptsächlich abhängig von der Löslichkeit des NLO-Materials und, falls die Anwendung der Mischung erfolgt, von der Löslichkeit des transparenten Polymeren. Geeignete Lösungsmittel sind neben Chloroform, N-Methylpyrrolidon, Diethylenglykoldiethylether, aliphatische Lösungsmittel oder auch Lösungsmittelgemische.

Als Trägermaterialien eignen sich im allgemeinen Glas, Siliziumwafer oder auch transparente Polymere, darunter Polymethylmethacrylat und Polystyrol.

Beispiele

Stellvertretend für die Synthese der Polyene werden im folgenden die Herstellung und die analytischen Daten von $C_{50}$-Astaxanthin gegeben. Die Synthese der höheren bzw. niedrigen Homologen basiert auf der gleichen Grundsynthese.

Beispiel 1

Synthese von $C_{50}$-Astaxanthin

Umsetzung

115,5 g (0,2 mol) $C_{15}$-Phosphoniumsalz (Formel 2) und 24,5 g (0,083 mol) Crocetindialdehyd (Formel 3)

wurden in 350 ml Methylenchlorid vorgelegt. Dazu wurden unter Kühlung tropfenweise 36,2 g (0,2 mol) 30 %ige methanolische Natriummethylatlösung gegeben und 4 h ohne Kühlung weitergerührt.

Abtrennung von Salz und Triphenylphosphinoxid

Zur Abtrennung von Salz und Triphenylphosphinoxid wurden 300 ml Wasser zugesetzt. Anschließend wurde die Methylenchloridphase abgetrennt und innerhalb von 30 min tropfenweise mit 300 ml Methanol versetzt. Gleichzeitig wurde das Methylenchlorid über eine Kolonne abdestilliert. Nach Zugabe von 80 ml Wasser wurden die gebildeten Kristalle abfiltriert, in 400 ml Methylenchlorid gewaschen und durch Lösungsmittelaustausch gegen 300 ml Methanol wieder ausgefällt.

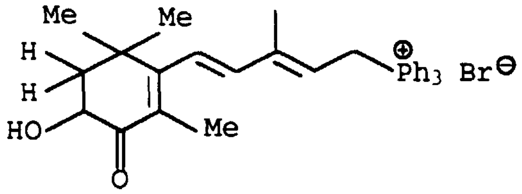

Formel 2

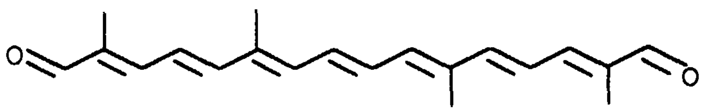

Formel 3

Isomerisierung

Die Kristalle wurden in 400 ml Methylenchlorid gelöst. Anschließend wurden 900 ml n-Heptan hinzugegeben und gleichzeitig das Methylenchlorid über eine Kolonne abdestilliert, die Suspension 16 h unter Rückfluß gekocht und dann die entstandenen Kristalle abgesaugt.

Reinigung

Das kristalline Produkt wurde in 400 ml Methylenchlorid gelöst, durch Lösungsmitteltausch gegen 900 ml Methanol wieder ausgefällt und nach Abkühlen auf 0°C abfiltriert. Nach der Trocknung wurden 43,4 g (59,8 % d. Th.) an kristallinem $C_{50}$-Astaxanthin erhalten.

Beispiel 2V (zum Vergleich)

$C_{30}$-Astaxanthin/Polymethylmethacrylatbeschichtung

In 5 g $CHCl_3$ wurden bei 25°C innerhalb von 30 min unter Rühren 60 mg Polymethylmethacrylat (z.B. Lucryl C 75 der Firma BASF) gelöst. Anschließend wurden 60 mg $C_{30}$-Astaxanthin (Tabelle 2) zugefügt und weitere 30 min nachgerührt. Man erhielt eine violette Lösung.

10 Tropfen dieser Lösung wurden innerhalb von 15 s auf ein mit 5000 $U \cdot min^{-1}$ rotierendes Glassubstrat getropft. Anschließend wurde noch 15 s abgeschleudert. Nach dieser Behandlung erhielt man beschichtete Glasplättchen, die im Vakuum 1 h bei 25°C getrocknet wurden.

Die physikalischen Daten der Beschichtung sind in Tabelle 1 zusammengestellt.

Beispiel 3V (zum Vergleich)

$C_{40}$-Astaxanthin/Polymethylmethacrylatbeschichtung

In Analogie zu Beispiel 2 wurden 60 mg Polymethylmethacrylat (z.B. Lucryl C 75 der Firma BASF) und 60 mg $C_{40}$-Astaxanthin (Tabelle 2) in 5 g Chloroform gelöst. Die anschließende Beschichtung mittels Spincoater ergab eine tiefviolette Schicht. Die physikalischen Meßdaten sind in Tabelle 1 wiedergegeben.

Beispiel 4

$C_{50}$-Astaxanthin/Polymethylmethacrylatbeschichtung

Ausgehend von $C_{50}$-Astaxanthin (Tabelle 2) wurden entsprechend den Versuchen 2 und 3 ebenfalls Beschichtungen auf Glassubstraten durchgeführt. (Ergebnisse s. Tabelle 1.)

Beispiel 5

$C_{60}$-Astaxanthin/Polymethylmethacrylatbeschichtung

In Analogie zu den vorangehenden Beispiele wurde auch N-Methylpyrrolidon (NMP) als Lösungsmittel zur Beschichtung eingesetzt. Die Lösung aus 90 mg Polymethylmethacrylat (z.B. Lucryl G 66 der Firma BASF), 90 mg $C_{60}$-Astaxanthin (Tabelle 2) und 7,5 g NMP führte nach dem Spincoaten ebenfalls zu homogenen, braun-violetten Filmen auf Glassubstraten.

Tabelle 1

| Physikalische Daten zu den Beispielen 2 - 5 | | | |
|---|---|---|---|
| Beispiel | d [nm] | $UV_{max}$ [um] | $x^3$ [esu] |
| 2V | 267 | 404 | $2{,}2 \cdot 10^{-12}$ |
| 3V | 420 | 479 | $8{,}7 \cdot 10^{-12}$ |
| 4 | 380 | 513 | $4{,}5 \cdot 10^{-11}$ |
| 5 | 138 | 531 | $1{,}2 \cdot 10^{-10}$ |
| Abkürzungen<br>d: Schichtdicke in nm<br>$UV_{max}$: Wellenlänge des Maximums der UV Absorption in nm<br>$x^3$: dielektrische Suszeptibilität 3. Ordnung in esu | | | |

Astaxanthine: Verbindungen der allgemeinen Formel 1 mit unterschiedlicher Anzahl an konjugierten Doppelbindungen entsprechend Tabelle 2

Tabelle 2
Definition der eingesetzten Polyene

| Astaxanthin | n in Formel |
|---|---|
| $C_{30}$- | 1 |
| $C_{40}$- | 2 |
| $C_{50}$- | 3 |
| $C_{60}$- | 4 |

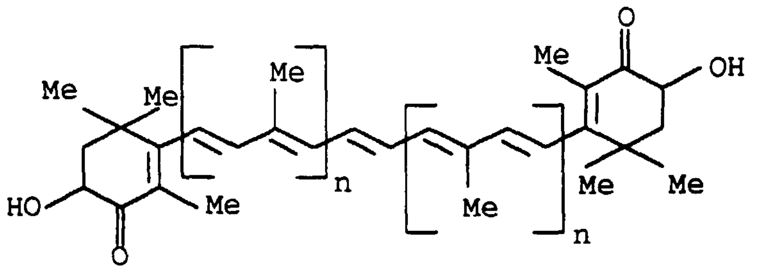

## Patentansprüche

1. Polyene der allgemeinen Formel I

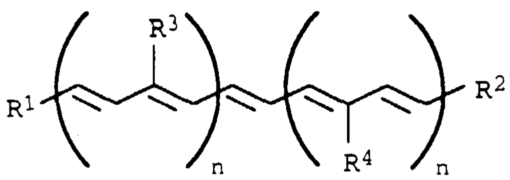

(I)

worin $R^1$ und $R^2$ gleich oder verschieden voneinander sein können und Wasserstoff, $C_6$- bis $C_{22}$-Aryl, $C_5$- bis $C_{10}$-Heteroaryl, $C_4$- bis $C_{30}$-Cycloalkyl, $C_4$- bis $C_{30}$-Cycloalkenyl, $C_6$- bis $C_{10}$-Cycloalkenon, wobei die vorgenannten Reste jeweils auch einen oder mehrere Substituenten ausgewählt aus der Gruppe der $C_1$- bis $C_{30}$-Alkyl-, $C_2$- bis $C_{30}$-Alkenyl-, $C_1$- bis $C_{30}$-Alkoxy-, $C_4$- bis $C_{30}$-Cycloalkyl-, $C_4$- bis $C_{30}$-Cycloalkenyl-, $C_6$- bis $C_{18}$-Arylreste, Halogen, -OH, -COOH, -CHO, $-COOR^5$, CN, $-NH_2$, $-NHR^6$, $-NR^7R^8$ und $NO_2$ haben können, -CHO, -COOH, $-COOR^9$ oder einen Rest der allgemeinen Formel

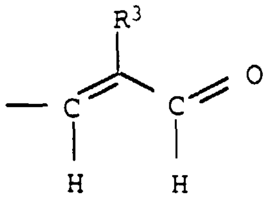

,

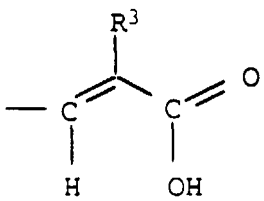

oder

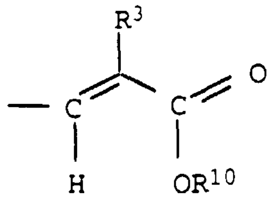

bedeuten und $R^3$ und $R^4$ gleich oder verschieden voneinander sein können und Wasserstoff, $C_1$- bis $C_{30}$-Alkyl, $C_1$- bis $C_{30}$-Alkoxy, -SH, -SSH, $-NH_2$, $-NR^{11}R^{12}$ bedeuten, wobei die Reste $R^5$ bis $R^{12}$ gleich oder verschieden und unabhängig voneinander $C_1$- bis $C_{30}$-Alkyl, $C_2$- bis $C_{30}$-Alkenyl, $C_4$- bis $C_{30}$-Cycloalkyl, $C_4$- bis $C_{30}$-Cycloalkenyl, $C_6$- bis $C_{18}$-Aryl sein können und n eine ganze Zahl von 3 bis 10 darstellt.

**2.** Polyene nach Anspruch 1, in denen die Reste $R^1$ and $R^2$

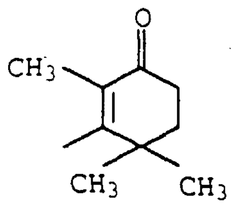

oder

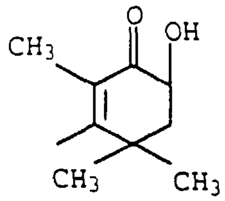

oder

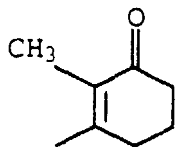

bedeuten.

**3.** Verfahren zur Herstellung von Polyenen nach Anspruch 1 oder 2, dadurch gekennzeichnet, daß man entsprechend mit $R^3$ und $R^4$ substituierte Alkene mit einer Anzahl von Doppelbindungen m < n und n eine ganze Zahl von 3 bis 10 darstellt, und einer oder zwei endständigen Aldehyd und/oder Ketogruppen einer Carbonylolefinierungsreaktion unterwirft.

**4.** Verfahren zur Herstellung von Polyenen nach Anspruch 3, dadurch gekennzeichnet, daß man Phosphoniumbromide der allgemeinen Formel II

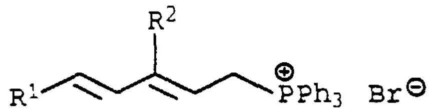

II

worin Ph Phenyl bedeutet in Gegenwart einer starken Base mit einem Alken mit zwei endständigen Aldehydgruppen der allgemeinen Formel III umsetzt

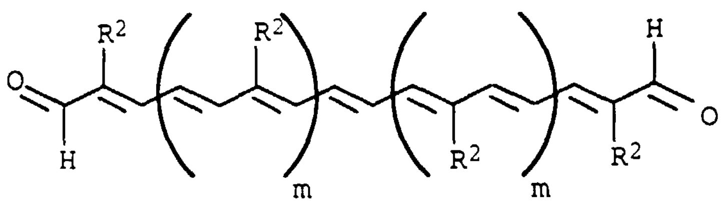

III

worin m eine ganze Zahl von 1 bis 4 ist.

**5.** Mischungen, enthaltend Polyene gemäß Anspruch 1 und transparente organische Polymere.

**6.** Lösungen, enthaltend Polyene gemäß Anspruch 1 oder Mischungen gemäß Anspruch 5.

**7.** Verwendung der Lösungen gemäß Anspruch 6 zum Beschichten von Trägermaterialien.

**8.** Beschichtete Trägermaterialien, enthaltend Polyene gemäß Anspruch 1.

**9.** Verwendung der beschichteten Trägermaterialien gemäß Anspruch 8 als optoelektrische Bauteile.

**10.** Verwendung der beschichteten Trägermaterialien nach Anspruch 8 als nichtlinear optische Bauteile.

**Claims**

1. A polyene of the general formula I

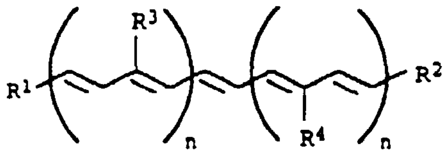
(I)

where $R^1$ and $R^2$ may be identical to or different from one another and are hydrogen, $C_6$- to $C_{22}$-aryl, $C_5$- to $C_{10}$-heteroaryl, $C_4$- to $C_{30}$-cycloalkyl, $C_4$- to $C_{30}$-cycloalkenyl or $C_6$- to $C_{10}$-cycloalkenone, where the abovementioned radicals may each also have one or more substituents selected from the group consisting of $C_1$- to $C_{30}$-alkyl, $C_2$- to $C_{30}$-alkenyl, $C_1$ to $C_{30}$-alkoxy, $C_4$- to $C_{30}$-cycloalkyl, $C_4$- to $C_{30}$-cycloalkenyl and $C_6$- to $C_{18}$-aryl radicals and halogen, -OH, -COOH, -CHO, $-COOR^5$, -CN, $-NH_2$, $-NHR^6$, $-NR^7R^8$ and $-NO_2$, or are -CHO, -COOH, $-COOR^9$ or a radical of the general formula

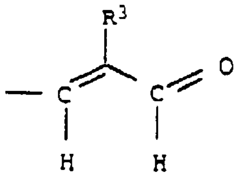 , 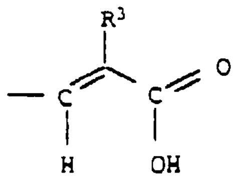 or 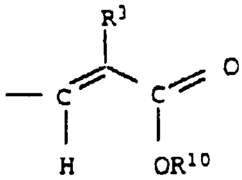

and $R^3$ and $R^4$ may be identical to or different from one another and are hydrogen, $C_1$- to $C_{30}$-alkyl, $C_1$-to $C_{30}$-alkoxy, -SH, -SSH, $-NH_2$ or $-NR^{11}R^{12}$, where the radicals $R^5$ to $R^{12}$ may be identical or different and are, independently of one another, $C_1$- to $C_{30}$-alkyl, $C_2$- to $C_{30}$-alkenyl, $C_4$- to $C_{30}$-cycloalkyl, $C_4$- to $C_{30}$-cycloalkenyl or $C_6$- to $C_{18}$-aryl, and n is an integer from 3 to 10.

2. A polyene as claimed in claim 1, where the radicals $R^1$ and $R^2$ are

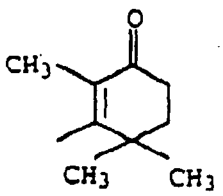 or 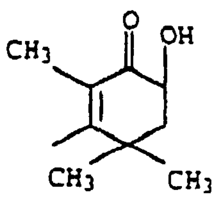 or 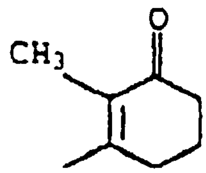

3. A process for the preparation of a polyene as claimed in claim 1 or 2, which comprises subjecting an alkene which is appropriately substituted by $R^3$ and $R^4$ and which has a number of double bonds m < n and n is an integer from 3 to 10, and one or two terminal aldehyde and/or keto groups, to a carbonyl olefination reaction.

4. A process for the preparation of a polyene as claimed in claim 3, which comprises reacting a phosphonium bromide of the general formula II

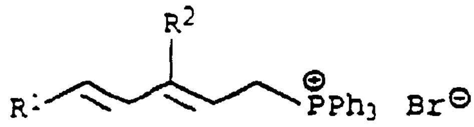

II

where Ph is phenyl, with an alkene having two terminal aldehyde groups, of the general formula III

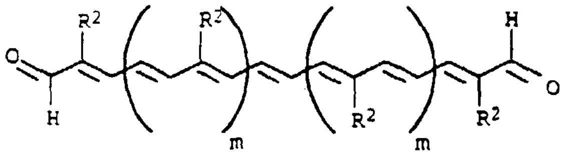

III

where m is an integer from 1 to 4, in the presence of a strong base.

**5.** A mixture comprising a polyene as claimed in claim 1 and a transparent organic polymer.

**6.** A solution comprising a polyene as claimed in claim 1 or a mixture as claimed in claim 5.

**7.** The use of a solution as claimed in claim 6 for coating support materials.

**8.** A coated support material comprising a polyene as claimed in claim 1.

**9.** The use of a coated support material as claimed in claim 8 as an opto-electrical component.

**10.** The use of a coated support material as claimed in claim 8 as a nonlinear optical component.

**Revendications**

**1.** Polyènes de formule générale I

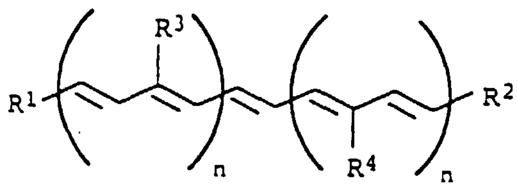

(I)

où $R^1$ et $R^2$ peuvent être identiques ou différents l'un de l'autre et représentent l'hydrogène ou un groupe aryle en $C_6$ à $C_{22}$, hétéroaryle en $C_5$ à $C_{10}$, cycloalkyle en $C_4$ à $C_{30}$, cycloalcényle en $C_4$ à $C_{30}$, cycloalcénone en $C_6$ à $C_{10}$, tandis que les restes susdits peuvent avoir à chaque fois également un ou plusieurs substituants choisis dans le groupe des restes alkyle en $C_1$ à $C_{30}$, alcényle en $C_2$ à $C_{30}$, alcoxy en $C_1$ à $C_{30}$, cycloalkyle en $C_4$ à $C_{30}$, cycloalcényle en $C_4$ à $C_{30}$, aryle en $C_6$ à $C_{18}$, halogéno, -OH, -COOH, -CHO, -COOR$^5$, -CN, -$NH_2$, -NHR$^6$, -NR$^7$R$^8$ et $NO_2$, et $R^1$ et $R^2$ représentent également un groupe -CHO, -COOH, -COOR$^9$ ou un reste de formule générale

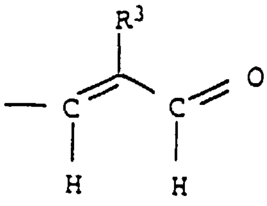 , 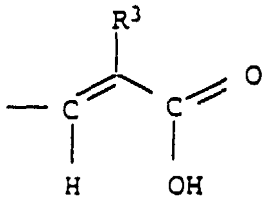 ou 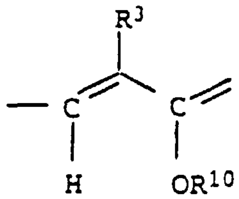

et $R^3$ et $R^4$ peuvent être identiques ou différents l'un de l'autre et représentent l'hydrogène ou un groupe alkyle en $C_1$ à $C_{30}$, alcoxy en $C_1$ à $C_{30}$, -SH, -SSH, $-NH_2$, $-NR^{11}R^{12}$, tandis que les restes $R^5$ à $R^{12}$ peuvent être identiques ou différents et représenter indépendamment les uns des autres des groupes alkyle en $C_1$ à $C_{30}$, alcényle en $C_2$ à $C_{30}$, cycloalkyle en $C_4$ à $C_{30}$, cycloalcényle en $C_4$ à $C_{30}$, aryle en $C_6$ à $C_{18}$, et n désigne un nombre entier de 3 à 10.

**2.** Polyènes selon la revendication 1, dans lesquels les restes $R^1$ et $R^2$ représentent

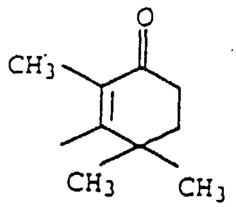 ou 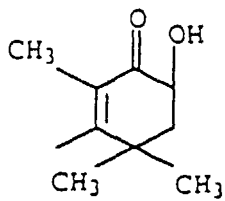 ou 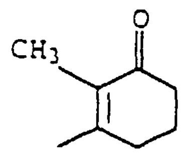

**3.** Procédé pour la préparation de polyènes selon la revendication 1 ou 2, caractérisé par le fait qu'on soumet à une réaction de carbonyloléfination des alcènes convenablement substitués par $R^3$ et $R^4$, ayant un nombre de doubles liaisons m < n, tandis que n représente un nombre entier de 3 à 10, et un ou deux groupes aldéhydiques et/ou cétoniques terminaux.

**4.** Procédé pour la préparation de polyènes selon la revendication 3, caractérisé par le fait qu'on fait réagir des bromures de phosphonium de formule générale II

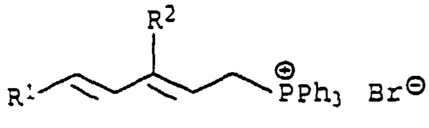

II

où Ph désigne un groupe phényle, en présence d'une base forte avec des alcènes ayant deux groupes aldéhydiques terminaux de formule générale III

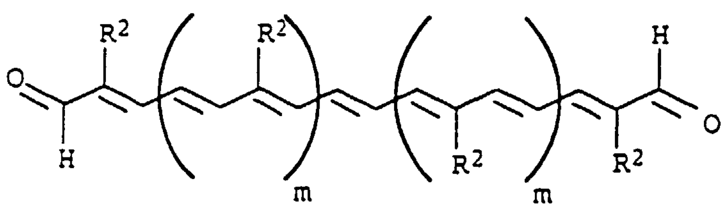

III

où m est un notre entier de 1 à 4.

**5.** Mélanges, contenant des polyènes selon la revendication 1 et des polymères organiques tansparents.

**6.** Solutions, contenant des polyènes selon la revendication 1 ou des mélanges selon la revendication 5.

**7.** Utilisation des solutions selon la revendication 6 pour le revêtement de matériaux supports.

**8.** Matériaux supports revêtus, contenant des polyènes selon la revendication 1.

**9.** Utilisation des matériaux supports revêtus selon la revendication 8 comme composants optoélectriques.

**10.** Utilisation des matériaux supports revêtus selon la revendication 8 comme composants d'optique non-linéaire.